# EUROPÄISCHE PATENTSCHRIFT

(11) **EP 2 364 288 B1**
(45) Veröffentlichungstag und Bekanntmachung des Hinweises auf die Patenterteilung: **11.06.2014**
(21) Anmeldenummer: 09752784.0
(22) Anmeldetag: 04.11.2009
(51) Int. Cl.: C07C 67/08, C07C 67/54, C07C 67/58, C07C 67/60, C07C 69/54, C08F 265/04, C08F 220/18

(54) **AUFARBEITUNGSVERFAHREN BEI DER HERSTELLUNG VON (METH)ACRYLSÄUREALKYLESTERN**
PROCESSING METHOD IN THE PRODUCTION OF (METH)ACRYLIC ACID ALKYL ESTERS
PROCÉDÉ DE RETRAITEMENT POUR LA PRODUCTION D'ESTERS ALKYLIQUES D'ACIDE (MÉTH)ACRYLIQUE

(30) Priorität: 04.12.2008 DE 102008060218
(43) Veröffentlichungstag der Anmeldung: 14.09.2011
(73) Patentinhaber: Evonik Degussa GmbH, 45128 Essen (DE)
(72) Erfinder: MOSLER, Jürgen, 45772 Marl (DE); KUPPINGER, Franz-Felix, 45768 Marl (DE)
(86) Internationale Anmeldenummer: PCT/EP2009/064569
(87) Internationale Veröffentlichungsnummer: WO 2010/063529

(56) Entgegenhaltungen:
- DE-A1- 19 935 453
- FR-A1- 2 884 514
- US-A1- 2008 015 384

## Beschreibung

Die vorliegende Erfindung betrifft ein Verfahren zur Herstellung einer hochreinen (Meth)Acrylsäurealkylester-Phase, die Verwendung einer Prozessabwasser-Phase, eine hochreine (Meth)Acrylsäurealkylester-Phase, ein Verfahren zur Herstellung eines auf (Meth)Acrylsäurealkylestern basierenden Polymers sowie das durch dieses Verfahren erhältliche, auf (Meth)Acrylsäurealkylestern basierende Polymer.

"(Meth)Acrylsäure" wird in diesem Text für die Verbindungen mit den Nomenklaturnamen "Methacrylsäure" und "Acrylsäure" verwendet. Von beiden Verbindungen ist die Acrylsäure sowohl im Zusammenhang mit dem erfindungsgemässen Verfahren als auch im Zusammenhang mit den erfindungsgemässen Polymeren bevorzugt.

Alkylester der (Meth)acrylsäure sind allgemein bekannt und beispielweise als Ausgangsmonomere zur Herstellung wässriger Polymerdispersionen von Bedeutung, die unter anderem als Klebstoffe Verwendung finden. In diesem Zusammenhang ist insbesondere n-Butylacrylat ein industriell besonders bedeutsames Monomer.

Verfahren zur Herstellung von (Meth)acrylsäurealkylestern durch Umsetzung von (Meth)acrylsäure mit 1 bis 5 Kohlenstoffatomen aufweisenden, einwertigen Alkoholen in homogener flüssiger Phase, bei erhöhter Temperatur und in Gegenwart Protonen liefernder Katalysatoren sind bekannt und beispielsweise in der DE-A-14 68 932, der DE-A-22 26 829 und der DE-A-22 52 334 beschrieben. Es handelt sich hierbei um typische Gleichgewichtsreaktionen, bei denen der Umsetzungsgrad der (Meth)acrylsäure und des jeweiligen Alkohols zum entsprechenden Ester durch die Gleichgewichtskonstante signifikant begrenzt ist. Um das Gleichgewicht auf die Seite des Reaktionsproduktes ((Meth)Acrylsäurealkylester) zu verschieben, wird daher das Wasser als Azeotrop zusammen mit nicht umgesetztem Alkohol mittels Destillation über Kopf aus dem Reaktionsgemisch entfernt. Ein solches Verfahren ist insbesondere in der DE-A-25 52 987 beschrieben.

Aus der auf diese Weise über Kopf abgezogenen, wässrigen Phase wird in einem Abscheider zumindest ein Teil des in dieser Phase enthaltenen Alkohols abgetrennt und in die Destillationskolonne zurückgeführt. Das den (Meth)Acrylsäurealkylester enthaltene Sumpfprodukt wird dann zunächst mittels einer Wasserwäsche von dem noch enthaltenem Veresterungskatalysator befreit und anschließend einer alkalischen Reaktivextraktion unterzogen, um insbesondere nicht umgesetzte (Meth)Acrylsäure als Alkali-Salz abzutrennen.

Das bei dieser alkalischen Reaktivextraktion erhaltene Sumpfprodukt wird üblicherweise mit der wässrigen Phase, welche nach Abtrennung zumindest eines Teils des Alkohols im Abscheider erhalten wird, vereinigt und dann einer Destillationskolonne zugeführt, in der ein weiterer Teil des noch enthaltenen, nicht umgesetzten Alkohols abgetrennt wird. Das in dieser Kolonne anfallende, wässrige Sumpfprodukt wird dann einer Verbrennungseinheit zugeführt.

FR 2 884 514 beschreibt ein Verfahren zur Herstellung von C1-C15 Alkyl(meth)acrylaten durch Direktveresterung von (Meth)acrylsäure mit dem entsprechenden Alkohol in Gegenwart von Schwefelsäure als Katalysator, wobei das Verfahren einen Verfahrensschritt der Hydrolysierung eines neutralisierten wässrigen Stroms aufweist, gefolgt von einer Extraktion von verbleibender (Meth)acrylsäure unter Verwendung eines Lösemittels, welches ganz oder teilweise aus dem Alkohol besteht, der bei der Reaktion eingesetzt wurde.

DE 199 35 453 offenbart ein kontinuierliches Verfahren zur Herstellung von Alkylestern der (Meth)acrylsäure, bei welchem eine Alkalisalze beinhaltende Nebenkomponentenphase mit Alkohol extrahiert wird, und das aus der Extraktion erhaltene, Alkalisalz-haltige Abwasser einer Kläranlage zugeführt wird.

Der Nachteil der aus dem Stand der Technik bekannten Verfahren zur Herstellung von (Meth)Acrylsäurealkylestern besteht jedoch unter anderem darin, dass sehr große Mengen an wässrigem Sumpfprodukt anfallen, die, da sich noch beachtliche Mengen an organischen Verunreinigungen enthält, verbrannt werden muss. Da die Entsorgung über eine Verbrennung dieses wässrigen Sumpfproduktes mitunter Primärenergiekosten in einer Höhe von mehr als 100 €/t verursacht und bei der Produktion von beispielsweise n-Butylacrylat teilweise mehr als 2 t/h an einem solchen wässrigen Sumpfprodukt anfallen, ist insbesondere die Entsorgung der Nebenkomponenten-Ströme bei der Herstellung von (Meth)Acrylsäurealkylestern sehr kostenintensiv. Darüber hinaus ist die Verbrennung des wässrigen Sumpfproduktes aufgrund der darin noch enthaltenen, organischen Nebenkomponenten auch nicht besonders umweltverträglich.

Der vorliegenden Erfindung lag die Aufgabe zugrunde, die sich aus dem Stand der Technik ergebenden Nachteile zu überwinden.

Insbesondere lag der vorliegenden Erfindung die Aufgabe zugrunde, ein Verfahren zur Herstellung von (Meth)Acrylsäurealkylestern anzugeben, welches im Vergleich zu den aus dem Stand der Technik bekannten Verfahren deutlich umweltverträglicher durchgeführt werden kann.

Auch lag der vorliegenden Erfindung die Aufgabe zugrunde, ein Verfahren zur Herstellung von (Meth)Acrylsäurealkylestern anzugeben, welches im Vergleich zu den aus dem Stand der Technik bekannten Verfahren insbesondere hinsichtlich der Entsorgung der Nebenkomponenten-Ströme kostengünstiger durchgeführt werden kann.

Einen Beitrag zur Lösung der eingangs genannten Aufgaben leistet ein Verfahren zur Herstellung einer hochreinen (Meth)Acrylsäurealkylester-Phase, beinhaltend die Verfahrensschritte:
I) Umsetzung einer Alkohol-Phase mit einer (Meth)Acrylsäure-Phase in Gegenwart eines Veresterungskatalysators unter Erhalt einer (Meth)Acrylsäurealkylester-Phase;
II) Destillative Abtrennung zumindest eines Teils des in der (Meth)Acrylsäurealkylester-Phase enthaltenen Wassers unter Erhalt
   - einer wasserarmen, den (Meth)Acrylsäurealkylester beinhaltenden (Metla)Acrylsäurealkylester-Phase, sowie
   - eines wässrigen Kopfproduktes;
III) Trennung des wässrigen Kopfproduktes in eine organische Phase und eine Prozessabwasser-Phase;
IV) weitere Aufreinigung der wasserarmen, den (Meth)Acrylsäurealkylester beinhaltenden (Meth)Acrylsäurealkylester-Phase durch ein Au.freinigungsverfahren beinhaltend einen alkalische Reaktivextraktions-Schritt unter Erhalt
   - einer aufgereinigten (Meth)Acrylsäurealkylester-Phase sowie
   - einer Alkali-Salze beinhaltenden Nebenkomponenten-Phase;
V) Befreien der aufgereinigten (Meth)Acrylsäurealkylester-Phase von weiteren Nebenprodukten unter Erhalt einer hochreinen (Meth)Acrylsäurealkylester-Phase;
   wobei mindestens eine der folgenden Alternativen i) oder ii), vorzugsweise i) und ii) zutrifft
   i) die Alkali-Salze beinhaltende Nebenkomponenten-Phase wird einer Verbrennungseinheit zugeführt, wobei die Alkali-Salze beinhaltende Nebenkomponenten-Phase vor der Zuführung in die Verbrennungseinheit mit maximal 50 Gew.-%/Stunde, besonders bevorzugt mit maximal 25 Gew.-%/Stunde und am meisten bevorzugt mit maximal 10 Gew.-%/Stunde, jeweils bezogen auf die pro Stunde anfallende Menge an Alkali-Salzen beinhaltende Nebenkomponenten-Phase, der Prozessabwasser-Phase vereinigt wird, wobei es am meisten bevorzugt ist, dass die Alkali-Salze beinhaltende Nebenkomponenten-Phase vor der Zufiihrung in die Verbrennungseinheit überhaupt nicht mit der Prozessabwasser-Phase vereinigt wird;
   ii) die Prozessabwasser- Phase wird, nach Abtrennung mindestens eines Teils des in der Prozessabwasser-Phase noch enthaltenen, nicht umgesetzten Alkohols, der Reinigung in einer Kläranlage zugeführt, wobei die Prozessabwasser-Phase vor der Zuführung in die Kläranlage mit maximal 50 Gew.-%/Stunde, besonders bevorzugt mit maximal 25 Gew.-%/Stunde und am meisten bevorzugt mit maximal 10 Gew.-%/Stunde, jeweils bezogen auf die pro Stunde anfallende Menge an Prozessabwasser-Phase, mit der die Alkali-Salze beinhaltenden Nebenkomponenten-Phase vereinigt wird, wobei es am meisten bevorzugt ist, dass die Prozessabwasser-Phase vor der Zuführung in die Kläranlage überhaupt nicht mit der die Alkali-Salze beinhaltenden Nebenkomponenten-Phase vereinigt wird.

Im Verfahrensschritt I) des erfindungsgemäßen Verfahrens wird zunächst eine Alkohol-Phase mit einer (Meth)Acrylsäure-Phase in Gegenwart eines Veresterungskatalysators unter Erhalt einer (Meth)Acrylsäurealkylester-Phase umgesetzt, wobei es insbesondere bevorzugt ist, das die Alkohol-Phase n-Butanol und die (Meth)Acrylsäure-Phase Acrylsäure beinhaltet (bei dem entstehenden (Meth)Acrylsäurealkylester handelt es sich daher vorzugsweise um n-Butylacrylat). Im Verfahrensschritt II) erfolgt dann die destillative Abtrennung zumindest eines Teils des in der (Meth)Acrylsäurealkylester-Phase enthaltenen Wassers (und auch mindestens eines Teils in der (Meth)Acrylsäurealkylester-Phase enthaltenen, nicht umgesetzten Alkohols) unter Erhalt einer wasserarmen, den (Meth)Acrylsäurealkylester beinhaltenden (Meth)Acrylsäurealkylester-Phase, sowie eines wässrigen Kopfproduktes.

Realisiert werden diese beiden Verfahrensstufen I) und 11) vorzugsweise dadurch, dass in einer Reaktionszone beinhaltend einen oder mehrere Reaktionsbereiche die Veresterungsreaktion durchgeführt wird und auf den einen oder die mehreren Reaktionsbereiche eine oder mehrere Rektifikationseinheiten aufgesetzt werden, mittels derer zumindest ein Teil des in der (Meth)Acrylsäurealkylester-Phase enthaltenen Wassers und auch mindestens ein Teil des in der (Meth)Acrylsäurealkylester-Phase enthaltenen, nicht umgesetzten Alkohols über Kopf abgetrennt wird. Ein solches Verfahren ist beispielsweise in der DE-A-196 04 252 beschrieben, deren Offenbarung hinsichtlich der Trennung von Veresterungsreaktion und destillativer Abtrennung des (Meth)Acrylsäurealkylesters hiermit als Referenz eingeführt wird.

Unter dem Begriff "Rektifikationseinheit" werden erfindungsgemäß vorzugsweise Vorrichtungen verstanden, in denen durch Wärmezufuhr aus zumindest einem Teil einer in der Rektifikationseinheit befindlichen, flüssigen Phase eine gasförmige Phase erzeugt wird, die innerhalb der Rektifikationseinheit aufsteigt und in Kontakt mit der abströmender flüssigen Phase steht. Der Begriff "Rektifikationseinheit" umfasst mithin auch einfache Destillationskolonnen. In der Regel handelt es sich hierbei jedoch um Rektifikationskolonnen, in denen Einbauten für den intensiven Kontakt zwischen flüssiger und gasförmiger Phase enthalten sind. Derartige Einbauten sind Böden, wie Glockenböden; Lochböden, insbesondere Dual-Flow-Böden, Schüttungen, Packungen oder dergleichen.

Im Zusammenhang mit dem Verfahrensschritt 1) ist es erfindungsgemäß besonders bevorzugt, dass die Alkohol-Phase zu mindestens 75 Gew.-%, noch mehr bevorzugt zu mindestens 90 Gew.-%, darüber hinaus bevorzugt zu mindestens 95 Gew.-% und am meisten bevorzugt zu mindestens 99 Gew.-%, jeweils bezogen auf das Gesamtgewicht der Alkoholphase, auf n-Butanol basiert, während die (Meth)Acrylsäure-Phase vorzugsweise zu mehr als 75 Gew.-%, noch mehr bevorzugt zu mehr als 90 Gew.-%, darüber hinaus bevorzugt zu mehr als 95 Gew.-%, darüber hinaus noch mehr bevorzugt zu mehr als 99 Gew.-% und am meisten bevorzugt zu mehr als 99,5 Gew.-%, jeweils bezogen auf das Gesamtgewicht der (Meth)Acrylsäure-Phase, auf Acrylsäure basiert.

Die Umsetzung der Alkohol-Phase mit der (Meth)Acrylsäure-Phase im Verfahrensschritt I) findet in einer Reaktionszone statt, welche gegebenenfalls aus mehreren Reaktionsbereichen bestehen kann, wobei es in diesem Fall vorteilhaft sein kann, diese mehreren Reaktionsbereiche zu kaskadieren. Der flüssige Austragsstrom eines Reaktionsbereiches bildet dabei den Zulauf des nachfolgenden Reaktionsbereiches. Dies kann mit Hilfe eines Überlaufes geschehen. Vorzugsweise werden dabei 2, 3 oder 4 Reaktionsbereiche, ganz besonders bevorzugt 4 Reaktionsbereiche kaskadiert. Wird mehr als ein Reaktionsbereich innerhalb ein und desselben Reaktors geschaffen (z. B. durch den Einsatz von Trennblechen), so kann die Anzahl der Reaktionsbereiche auch größer 4 sein. Im Falle von mehreren Reaktionsbereichen werden die Brüden der Reaktionsbereiche einer gemeinsamen Rektifikationseinheit, beispielsweise einer gemeinsamen Rektifikationskolonne, zugeführt, deren flüssiger Ablauf vorteilhafterweise in den ersten Reaktionsbereich gelangt.

Die Trennung der Veresterungsreaktion im Verfahrensschritt I) und der destillative Abtrennung des Alkylesters der (Meth)acrylsäure im Verfahrensschritt II) erlaubt mildere Reaktionsbedingungen. Die Reaktion kann in allen Reaktionsbereichen bei einem Druck von 100 mbar bis Atmosphärendruck, vorzugsweise bei 200 bis 700 mbar, besonders bevorzugt bei 300 bis 450 mbar Kopfdruck (Rektifikationskolonne) und einer Temperatur von 90°C bis 115°C betrieben werden. Der Druck kann dabei in allen Reaktionsbereichen gleich sein, es kann jedoch auch vorteilhaft sein, die Veresterungsreaktionen in den einzelnen Reaktionsbereichen bei unterschiedlichen Druckbedingungen durchzuführen, und, im Falle nur einer einzigen, aufgesetzten Rektifikationseinheit, die Brüden der Reaktionsbereiche in unterschiedliche Höhen der Rektifikationseinheit einzuspeisen.

Als Veresterungskatalysator im Verfahrensschritt I) können alle dem Fachmann bekannten Katalysatoren eingesetzt werden, welche die Umsetzung von (Meth)Acrylsäure mit Alkoholen, insbesondere die Umsetzung von Acrylsäure mit n-Butanol, katalysieren, wobei der Einsatz von katalytisch wirksamen Säuren besonders bevorzugt, der Einsatz von Sulfonsäuren oder Schwefelsäure noch mehr bevorzugt und der Einsatz von p-Toluolsulfonsäure am meisten bevorzugt ist. Es ist in diesem Zusammenhang erfindungsgemäß weiterhin bevorzugt, dass der Gehalt an katalytisch wirksamer Säure, am meisten bevorzugt von p-Toluolsulfonsäure, im ersten Reaktionsbereich, bezogen auf das darin enthaltene Reaktionsgemisch, in einem Bereich von 0,1 bis 10 Gew.-%, bevorzugt in einem Bereich von 0,1 bis 6 Gew.-% liegt. Die Gesamtverweilzeit der Reaktanden in der Reaktionszone beträgt in der Regel 0,25 bis 15 Stunden, vorzugsweise 1 bis 7 Stunden, besonders bevorzugt 2 bis 5 Stunden. In aufeinander folgenden Bereichen nimmt sie vorzugsweise ab.

Im Verfahrensschritt IV) des erfindungsgemäßen Verfahrens wird die wasserarme, den (Meth)Acrylsäurealkylester beinhaltenden (Meth)Acrylsäurealkylester-Phase, welche in der Reaktionszone zurückbleibt, durch ein Aufreinigungsverfahren beinhaltend einen alkalischen Reaktivextraktions-Schritt unter Erhalt einer aufgereinigten (Metla)Acrylsäurealkylester-Phase sowie einer Alkali-Salze beinhaltenden Nebenkomponenten-Phase aufgetrennt.

Dabei ist es jedoch bevorzugt, vor der alkalischen Reaktivextraktion die wasserarme, den (Meth)Acrylsäurealkylester beinhaltenden (Meth)Acrylsäurealkylester-Phase zunächst mittels einer Wasserwäsche von zumindest einem Teil des noch in dieser Phase enthaltenen Katalysators zu befreien. Dazu wird die die wasserarme, den (Meth)Acrylsäurealkylester beinhaltende (Meth)Acrylsäurealkylester-Phase aus der Reaktionszone ausgetragen und in den unteren Bereich, vorzugsweise in das untere Drittel, noch mehr bevorzugt in das untere Viertel, einer Extrakationseinheit (nachfolgend - Extraktionseinheit A - genannt) zugeführt, in deren oberen Bereich, vorzugsweise in deren oberes Drittel, noch mehr bevorzugt in deren oberes Viertel, Wasser eingespeist wird, so dass das Wasser die in dieser Extraktionseinheit A aufsteigende organische Flüssigphase im Gegenstrom wäscht, wobei vorzugsweise sichergestellt wird, dass das Wasser in Form von Tropfen durch die kontinuierliche, organische Phase hindurch fällt. Am Kopf der Extraktionseinheit A wird auf diese Weise eine wasser- und katalysatorarme, den (Meth)Acrylsäurealkylester beinhaltende (Meth)Acrylsäure-alkylester-Phase erhalten, die dann der alkalischen Reaktivextraktion gemäß Verfahrensschritt IV) unterzogen wird. Die Extraktionseinheit A, in der mittels Wasserwäsche der Katalysator abgetrennt wird, wird bevorzugt bei Normaldruck und bei einer Temperatur in einem Bereich von 30 °C bis 60 °C betrieben. Die im unteren Teil dieser Extraktionseinheit A erhaltene wässrige Phase, welche große Mengen des Katalysators enthält, wird vorzugsweise in die Reaktionszone (sofern diese aus mehreren Reaktionsbereichen besteht, vorzugsweise in denjenigen Reaktionsbereich, in den auch die Alkohol- und die (Meth)Acrylsäure-Phase eingespeist werden) zurückgeführt.

Die am Kopf dieser Extraktionseinheit A erhaltene, wasser- und katalysatorarme, den (Meth)Acrylsäurealkylester beinhaltende (Meth)Acrylsäurealkylester-Phase wird dann einer alkalischen Reaktivextraktion unterzogen. Dazu wird die die wasser- und katalysatorarme, den (Meth)Acrylsäurealkylester beinhaltende (Meth)Acrylsäurealkylester-Phase aus dem Kopf der Extraktionseinheit A in den unteren Bereich, vorzugsweise in das untere Drittel, noch mehr bevorzugt in das untere Viertel, einer weiteren Extraktionseinheit (nachfolgend - Extraktionseinheit B - genannt) zugeführt, bei der vorzugsweise in den mitleren Bereich eine wässrige Alkalilösung, besonders bevorzugt Natronlauge, eingespeist wird, so dass die wässrige Alkalilösung die in dieser Extraktionseinheit B aufsteigende organische Flüssigphase im Gegenstrom wäscht und die in dieser Phase insbesondere noch enthaltenen Reste an Katalysator und nicht umgesetzter (Meth)Acrylsäure in Form ihrer Alkali-Salze in der wässrigen Lösung bindet. Dabei wird sichergestellt, dass die wässrige Alkalilösung in Form vereinzelter Tropfen durch die kontinuierliche, organische Flüssigphase hindurch fällt. Die Konzentration dieser wässrigen Alkalilösung liegt vorzugsweise in einem Bereich von 1 bis 50 Gew.-%, besonders bevorzugt in einem Bereich von 15 bis 40 Gew.-%. Ganz besonders vorteilhaft ist die Verwendung einer etwa 25 gew.-%igen Natriumhydroxidlösung, wobei die Menge der zugegebenen, wässrigen Natriumhydroxidlösung durch pH-Messung überwacht wird und sich nach dem Äquivalenzpunkt des Natrium(meth)acrylates richtet. Weiterhin ist es vorteilhaft, auch in dieser Extraktionseinheit B in den oberen Bereich, vorzugsweise in deren oberes Drittel, noch mehr bevorzugt in deren oberes Viertel, Wasser einzuspeisen. Die Extraktionseinheit B, in der mittels Laugenwäsche Verunreinigungen abgetrennt werden, wird ebenfalls bevorzugt bei Normaldruck und bei einer Temperatur in einem Bereich von 30 °C bis 60 °C betrieben.

Am Kopf der Extraktionseinheit B wird auf diese Weise eine aufgereinigte (Meth)Acrylsäurealkylester-Phase erhalten, während im unteren Bereich eine Alkali-Salze beinhaltende Nebenkomponenten-Phase zurückbehalten wird. Dabei ist es erfindungsgemäß besonders bevorzugt, dass die aufgereinigte (Meth)Acrylsäure-alkylester-Phase zu mindestens 77,5 Gew.-%, besonders bevorzugt zu mindestens 80 Gew.-% und am meisten bevorzugt zu mindestens 84,7 Gew.-%, jeweils bezogen auf das Gesamtgewicht der aufgereinigten (Meth)Acrylsäurealkylester-Phase, auf dem (Meth)Acrylsäurealkylester basiert.

Die im Verfahrensschritt IV) erhaltene; aufgereinigte (Meth)Acrylsaurealkylester-Phase wird dann in einem weiteren Verfahrensschritt V) von weiteren Nebenprodukten (Leicht- und Hochsieder, also Verbindungen, deren Siedepunkt niedriger oder höher als der des (Meth)Acrylsäurealkylesters ist) unter Erhalt einer hochreinen (Meth)Acrylsäurealkylester-Phase befreit, wobei diese weitere Aufreinigung vorzugsweise durch einen oder mehrere weitere(n) Destillationsschritt(e), vorzugsweise in einer oder mehreren weitere(n) Rektifikationseinheit(en), erfolgt. Die auf diese Weise erhaltene, hochreine (Meth)Acrylsäurealkylester-Phase basiert vorzugsweise zu mindestens 90 Gew.-%, besonders bevorzugt zu mindestens 95 Gew.-% und am meisten bevorzugt zu mindestens 99,7 Gew.-%, jeweils bezogen auf das Gesamtgewicht der hochreinen (Meth)Acrylsäurealkylester-Phase, auf dem (Meth)Acrylsäurealkylester.

Das im Verfahrensschritt II) am Kopf der Rektifikationseinheit erhaltene, wässrige Kopfprodukt, welches noch beachtliche Mengen an nicht umgesetztem Alkohol enthält, wird im Verfahrensschritt III) vorzugsweise mittels Kondensation in zwei Phasen, eine organische, überwiegend aus dem nicht umgesetzten Alkohol bestehende Phase und eine wässrige, überwiegend aus Wasser bestehende Phase (nachfolgend - Prozessabwasser-Phase - genannt) getrennt, wobei es erfindungsgemäß bevorzugt ist, die organische Phase zur Rektifikationseinheit zurückzuführen. Für diese Trennung der wässrigen von der organischen Phase können alle dem Fachmann bekannten Trennvorrichtungen, in denen eine Trennung von zwei miteinander in Kontakt stehenden, ein Zweiphasensystem bildenden, flüssigen Phasen möglich ist.

In diesem Zusammenhang ist es bevorzugt, dass das erfindungsgemäße Verfahren kontinuierlich betrieben wird, wobei der Reaktionszone kontinuierlich die (Meth)Acrylsäure-Phase, die Alkohol-Phase sowie frischer Katalysator zugesetzt wird und kontinuierlich eine Prozessabwasser-Phase, eine aufgereinigte (Meth)Acrylsäureester-Phase sowie eine die Alkali-Salze beinhaltende Nebenkomponenten-Phase erhalten wird. Die Prozessabwasser-Phase fällt dabei vorzugsweise in einer Menge in einem Bereich von 1.000 bis 3.000 t/h (t/h = Tonne pro Stunde), besonders bevorzugt in einem Bereich von 1.500 bis 2.500 t/h und meisten bevorzugt in einem Bereich von 1.750 bis 2.000 t/h an, während die die Alkali-Salze beinhaltende Nebenkomponenten-Phase vorzugsweise in einer Menge in einem Bereich von 100 bis 1.000 t/h, besonders bevorzugt in einem Bereich von 250 bis 850 t/h und meisten bevorzugt in einem Bereich von 350 bis 650 t/h anfällt.

Weiterhin ist es erfindungsgemäß bevorzugt, dass die wasserarme (Meth)Acrylsäurealkylester-Phase, besonders bevorzugt die wasserarme n-Butylacrylat-Phase, vor der alkalischen Reaktivextraktion und vorzugsweise nach der Wasserwäsche
(α1) 70 bis 90 Gew.-%, besonders bevorzugt 75 bis 85 Gew.-% und am meisten bevorzugt 80 bis 85 Gew.-% des (Meth)Acrylsäurealkylesters,
(α2) 5 bis 20 Gew.-% besonders bevorzugt 6,5 bis 15 Gew.-% und am meisten bevorzugt 8 bis 10 Gew.-% des nicht umgesetzten Alkohols,
(α3) 0,1 bis 5 Gew.-% besonders bevorzugt 0,5 bis 2 Gew.-% und am meisten bevorzugt 0,75 bis 1,25 Gew.-% der nicht umgesetzten (Meth)Acrylsäure,
(α4) 0,1 bis 10 Gew.-%, besonders bevorzugt 1 bis 8 Gew.-% und am meisten bevorzugt 2 bis 5 Gew.-% Wasser, sowie
(α5) 1 bis 10 Gew.-% besonders bevorzugt 2 bis 8 Gew.-% und am meisten bevorzugt 4 bis 6 Gew.-% von den Komponenten (α1) bis (α4) verschiedene Verunreinigungen,
beinhaltet, wobei die Summe der Komponenten (α1) bis (α5) 100 Gew.-% beträgt. Zu den von den Komponenten (α1) bis (α4) verschiedene Verunreinigungen gehören im Falle der n-Butylacrylat-Herstellung insbesondere β-Buthoxybutylpropionat, Acryloxybutylpropionat, Dibutylether sowie, im Falle eines Einsatzes von p-Toluolsulfonsäure als Katalysator, p-Toluolsulfonsäure.

Auch ist es erfindungsgemäß bevorzugt, dass die Alkali-Salze beinhaltende Nebenkomponenten-Phase
(β1) 60 bis 90 Gew.-%, besonders bevorzugt 70 bis 85 Gew.-% und am meisten bevorzugt 77,5 bis 82,5 Gew.-% Wasser,
(β2) 0,1 bis 5 Gew.-%, besonders bevorzugt 0,5 bis 3 Gew.-% und am meisten bevorzugt 0,75 bis 1,5 Gew.-% des nicht umgesetzten Alkohols,
(β3) 5 bis 30 Gew.-%, besonders bevorzugt 7,5 bis 20 Gew.-% und am meisten bevorzugt 10 bis 15 Gew.-% des Alkali-Salzes der nicht umgesetzten (Meth)Acrylsäure,
(β4) 0,1 bis 10 Gew.-%, besonders bevorzugt 1 bis 8 Gew.-% und am meisten bevorzugt 3 bis 6 Gew.-% von den Komponenten (β1) bis (β3) verschiedene Verunreinigungen,
beinhaltet, wobei die Summe der Komponenten (β1) bis (β4) 100 Gew.-% beträgt. Zu den von den Komponenten (β1) bis (β3) verschiedenen Verunreinigungen gehören im Falle der n-Butylacrylat-Herstellung insbesondere das zur Reaktivextraktion eingesetzte Alkalisalz, vorzugsweise NaOH, sowie, im Falle eines Einsatzes von p-Toluolsulfonsäure als Katalysator, das entsprechende Alkalisalz der p-Toluolsulfonsäure.

Es ist nun erfindungsgemäß ganz besonders bevorzugt, dass mindestens eine der folgenden Alternativen i) oder ii), vorzugsweise i) und ii) zutrifft:
i) die Alkali-Salze beinhaltende Nebenkomponenten-Phase wird einer Verbrennungseinheit zugeführt, wobei die Alkali-Salze beinhaltende Nebenkomponenten-Phase vor der Zuführung in die Verbrennungseinheit nicht mit der Prozessabwasser-Phase vereinigt wird;
ii) die Prozessabwasser-Phase wird, nach Abtrennung mindestens eines Teils des in der Prozessabwasser-Phase noch enthaltenen, nicht umgesetzten Alkohols, der Reinigung in einer Kläranlage zugeführt, wobei die Prozessabwasser-Phase vor der Zuführung in die Kläranlage nicht mit der die Alkali-Salze beinhaltende Nebenkomponenten-Phase vereinigt wird.

Gemäß Alternative i) wird die Alkali-Salze beinhaltende Nebenkomponenten-Phase einer Verbrennungseinheit zugeführt. Dabei ist es gemäß einer besonderen Ausgestaltung des erfindungsgemäßen Verfahrens bevorzugt, dass aus der Alkali-Salze beinhaltenden Nebenkomponenten-Phase vor deren Zuführung in die Verbrennungseinheit mindestens ein Teil des in dieser Nebenkomponenten-Phase noch enthaltenen Wassers unter Erhalt einer wasserarmen, Alkali-Salze beinhaltenden Nebenkomponenten-Phase abgetrennt wird, wobei diese Abtrennung vorzugsweise durch einfaches Verdampfen, gegebenenfalls gefolgt von einer Kondensation des verdampften Wassers, erfolgt. Denkbar ist hingegen auch eine Abtrennung des Wassers durch Extraktion.

In diesem Zusammenhang ist es weiterhin bevorzugt, aus der Alkali-Salze beinhaltenden Nebenkomponenten-Phase oder aus der wasserarmen, Alkali-Salze beinhaltenden Nebenkomponenten-Phase, besonders bevorzugt aus der wasserarmen, Alkali-Salze beinhaltenden Nebenkomponenten-Phase, mindestens einen Teil des in dieser Nebenkomponenten-Phase noch enthaltenen, nicht umgesetzten Alkohols und gegebenenfalls des ebenfalls noch enthaltenen Wassers weiter abzutrennen, wobei dieses Abtrennen vorzugsweise durch Elektrodialyse, durch Umkehrosmose oder eine Kombination dieser Trennverfahren erfolgt.

Die Umkehrosmose - auch Reverseosmose oder Hyperfiltration genannt - ist eine Druckfiltration an einer semipermeablen Membran, bei der insbesondere ein Druck benutzt wird, um den natürlichen Osmose-Prozess umzukehren. Der anzuwendende Druck muss dabei größer sein als der Druck, der durch das osmotische Verlangen zum Konzentrationsausgleich entstehen würde. Die osmotische Membran, die nur die Trägerflüssigkeit (Solvent) durchlässt und die gelösten Stoffe (Solute) zurückhält, muss derartig ausgelegt sein, diesen oft hohen Druck standzuhalten. Wenn der Druckunterschied das osmotische Gefälle mehr als ausgleicht, passen die Solventmoleküle wie bei einem Filter durch die Membran, während die Verunreinigungsmoleküle zurückgehalten werden. Der osmotische Druck steigt mit zunehmendem Konzentrationsunterschied und kommt zum Stillstand, wenn der natürliche osmotische Druck gleich dem vorgegebenen Druck ist. Bei einer kontinuierlichen Verfahrensweise wird das Konzentrat stetig abgeführt. Es können Durchflussmembranen verwendet werden. Die Alkali-Salze beinhaltende Nebenkomponenten-Phase oder aber die wasserarme, Alkali-Salze beinhaltende Nebenkomponenten-Phase werden dabei unter Drücken von in der Regel 2 bis 15 MPa gegen eine Porenmembran, vorzugsweise aus Cellusloseacetat oder Polyamid, gepresst, wobei Wasser und/oder nicht umgesetzter Alkohol, nicht aber die in diesem Lösungsmittelsystem gelösten Alkali-Salze durch die Poren der Membran hindurch treten können. Geeignete Membranmaterialien und Vorrichtungen können unter anderem dem Kapitel 10.7 "Permeation" in "Grundoperationen Chemischer Verfahrenstechnik", Wilhelm R. A. Vauck und Hermann A. Müller, WILEY-VCH-Verlag, 11. überarbeitete und erweiterte Auflage, 2000, entnommen werden.

Bei einer Elektroosmose stellt sich in einem Gleichgewichtszustand der elektroosmotische Druck ein, mit dem ebenso eine Trennung der Alkali-Salze vom Wasser und/oder vom nicht umgesetzten Alkohol erreicht werden kann. Bei der E-lektrosomose werden ebenfalls Wasser und/oder nicht umgesetzter Alkohol über eine semipermeable Membran aus einer wässrigen Zusammensetzung selektiv abgetrennt, wobei jedoch im Gegensatz zur Umkehrosmose das Wasser nicht über einen Überdruck, sondern durch das Anlegen einer elektrischen Spannung von in der Regel 6 bis 20 V durch die semipermeable Membran getrieben wird.

Der abgetrennte Alkohol kann gegebenenfalls in den Verfahrensschritt I) oder II), vorzugsweise II) zurückgeführt werden.

Gemäß der Alternative ii) wird die Prozessabwasser-Phase, nach Abtrennung mindestens eines Teils des in der Prozessabwasser-Phase noch enthaltenen, nicht umgesetzten Alkohols, der Reinigung in einer Kläranlage zugeführt. Die Abtrennung des noch in der Prozessabwasser-Phase enthaltenen Alkohols erfolgt dabei vorzugsweise durch destillative Trennverfahren, besonders bevorzugt in einer weiteren Rektifikationseinheit, wobei der auf diese Weise abgetrennte Alkohol vorzugsweise ebenfalls in den Verfahrenschritt I) oder II), besonders bevorzugt in den Verfahrensschritt II), zurückgeführt wird.

Einen Beitrag zur Lösung der eingangs genannten Aufgaben leistet weiterhin die Verwendung einer Prozessabwasser-Phase, erhalten in Verfahrensschritt III) des vorstehend beschriebenen Verfahrens, besonders bevorzugt jedoch einer Prozessabwasser-Phase, welche in Verfahrensschritt III) des vorstehend beschriebenen Verfahrens erhalten wurde und aus welcher der in dieser Prozessabwasser-Phase noch enthaltene Alkohol, wie ebenfalls vorstehend beschrieben, abgetrennt wurde, als wässrige Ausgangsphase in einer Kläranlage. Dabei ist es insbesondere bevorzugt, dass diese Prozessabwasser-Phase ohne vorherige Vereinigung mit der Alkali-Salze beinhaltenden Nebenkomponenten-Phase als wässrige Ausgangsphase in einer Kläranlage eingesetzt wird.

Einen Beitrag zur Lösung der eingangs genannten Aufgaben leistet auch eine hochreine (Meth)Acrylsäurealkylester-Phase, besonders bevorzugt eine hochreine n-Butylacrylat-Phase, erhältlich durch das vorstehend beschriebene Verfahren.

Einen Beitrag zur Lösung der eingangs genannten Aufgaben leistet auch ein Verfahren zur Herstellung eines auf (Meth)Acrylsäurealkylestern, besonders bevorzugt auf n-Butylacrylat, basierenden Polymers, beinhaltend die Verfahrensschritte
(a) Herstellung einer hochreinen (Meth)Acrylsäurealkylester-Phase, besonders bevorzugt einer n-Butylacrylat-Phase, durch das vorstehend beschriebene Verfahren;
(b) radikalische Polymerisation des (Meth)Acrylsäurealkylesters, besonders bevorzugt des n-Butylacrylates.

Einen Beitrag zur Lösung der eingangs genannten Aufgaben leistet weiterhin das durch das vorstehend beschriebene Verfahren erhältliche, auf (Meth)Acrylsäurealkylestem, besonders auf n-Butylacrylat, basierende Polymer.

Die Erfindung wird nun anhand nicht limitierender Figuren weiter erläutert.

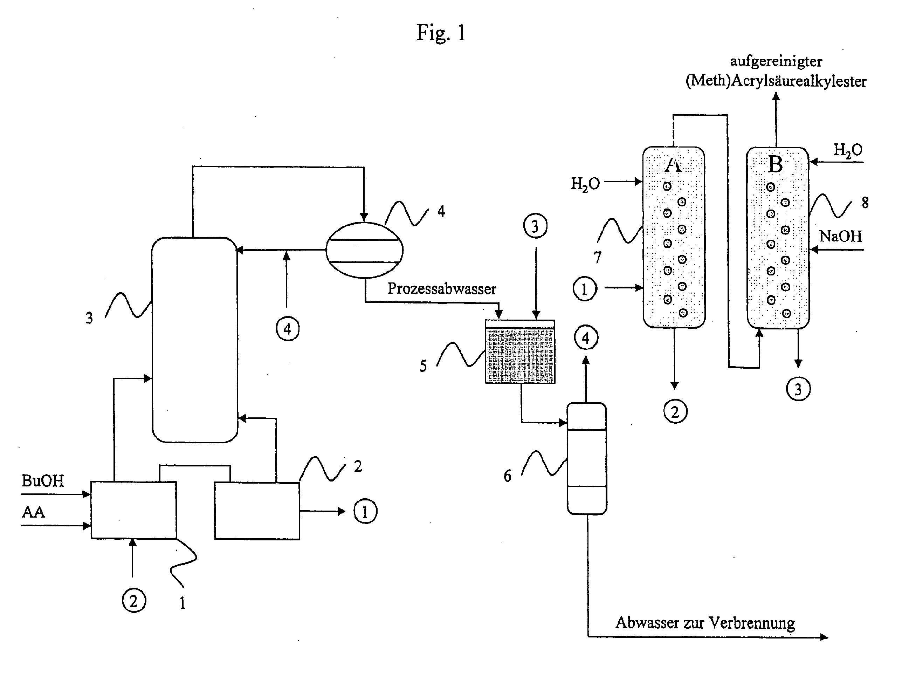

Es zeigt die Figur 1 ein herkömmliches Verfahren zur Herstellung eines (Meth)Acrylsäurealkylesters (n-Butylacrylat).

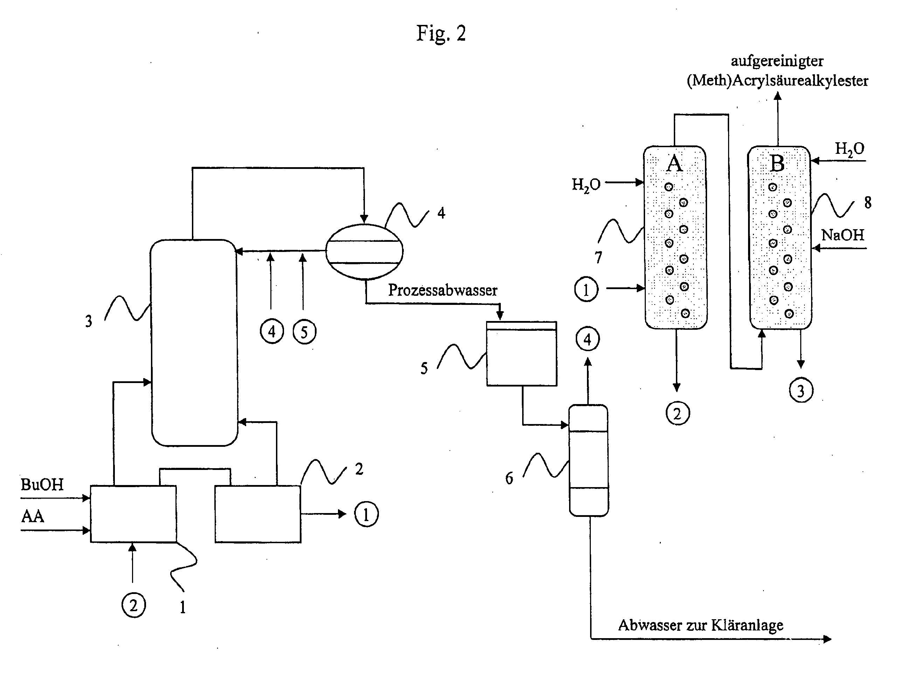

Es zeigt die Figur 2 das erfindungsgemäße Verfahren zur Herstellung eines (Meth)Acrylsäurealkylesters (n-Butylacrylat).

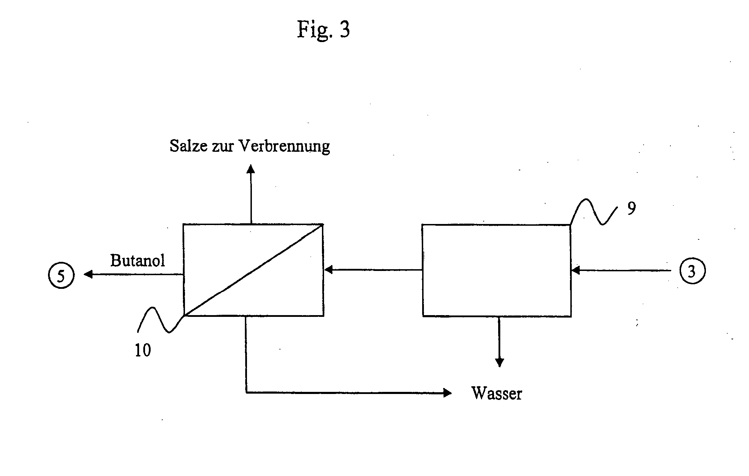

Es zeigt die Figur 3 eine erfindungsgemäß bevorzugte Behandlung des in der Rektifikationseinheit C erhaltenen Sumpfproduktes.

Gemäß der Figur 1 werden in einem herkömmlichen Verfahren zur Herstellung eines (Meth)Acrylsäurealkylesters in einer Reaktionszone, beispielsweise umfassend die beiden Reaktionsbereiche 1 und 2, n-Butanol (BuOH) und Acrylsäure (AA) zu n-Butylacrylat umgesetzt. Die eingesetzte Acrylsäure hat dabei vorzugsweise eine Reinheit von mindestens 75 Gew.-%, noch mehr bevorzugt mindestens 90 Gew.-%, darüber hinaus bevorzugt mindestens 95 Gew.-% und am meisten bevorzugt mindestens 99 Gew.-%. Der flüssige Austragsstrom des Reaktionsbereiches 1 bildet dabei den Zulauf des nachfolgenden Reaktionsbereiches 2 und gelangt über einen Überlaufes in den Reaktionsbereich 2. Aufgesetzt auf die Reaktionszone ist die Rektifikationseinheit 3, in welche die Brüden der Reaktionsbereiche 1 und 2 eingespeist werden. In dieser Rektifikationseinheit wird über Kopf ein wässriges Produkt, welches neben Wasser vor allem auch n-Butanol enthält, abgezogen und in einem Abscheider 3 kondensiert. Dabei bildet sich eine organische, im Wesentlichen aus dem n-Butanol bestehende Phase und eine im Wesentlichen aus Wasser bestehende Prozessabwasser-Phase, welche jedoch immer noch n-Butanol als Verunreinigung enthält. Die organische, im Wesentlichen aus n-Butanol bestehende Phase wird in die Rektifikationseinheit 3 zurückgeführt, während die Prozessabwasser-Phase in einen Sammelbehälter 5 gegeben wird.

Das in der Reaktionszone zurückbehaltene, das n-Butylacrylat enthaltene Reaktionsgemisch (①) wird zunächst einer Wasserwäsche in einer ersten Extraktionseinheit 7 ( = Extraktionseinheit A im Sinne der vorangegangenen Beschreibung) zugeführt, um vor allem den Katalysator (p-Toluolsulfonsäure) abzutrennen. Das Sumpfprodukt dieser Extraktionseinheit A (②), welches große Mengen an p-Toluolsulfonsäure enthält, wird in den Reaktionsbereich 1 zurückgeführt. Das Kopfprodukt dieser Extraktionseinheit (im hauptsächlich bestehend aus n-Butylacrylat) wird sodann einer alkalischen Reaktivextraktion in der weiteren Extraktionseinheit 8 ( = Extraktionseinheit B im Sinne der vorangegangenen Beschreibung) zugeführt, in der noch immer enthaltener Katalysator, nicht umgesetzte Acrylsäure sowie weitere Nebenprodukte in Form ihrer Alkali-Salze abgetrennt werden. Im Sumpf dieser Extraktionseinheit 8 wird eine Alkali-Salze beinhaltenden Nebenkomponenten-Phase (③) erhalten, welche mit der Prozessabwasser-Phase im Sammelbehälter 5 vereinigt wird. Das den Alkylester beinhaltende Kopfprodukt der Extraktionseinheit B wird in weiteren Destillationsschritten unter Erhalt einer hochreinen n-Butylacrylat-Phase weiter aufgereinigt (nicht gezeigt).

Aus der im Sammelbehälter 5 erhaltenen Zusammensetzug wird sodann mittels einer weiteren Rektifikationseinheit 6 noch enthaltenes n-Butanol abgetrennt (④), welches ebenfalls in die Rektifikationseinheit 3 zurückgeführt wird. Die in der Rektifikationseinheit 6 als Sumpfprodukt zurückbleibende, wässrige Phase wird sodann einer Verbrennungseinheit zugeführt.

Gemäß dem in Figur 2 gezeigten, erfindungsgemäßen Verfahren wird das in der Rektifikationskolonne 8 zurückbehaltende, Alkali-Salze beinhaltenden Nebenkomponenten-Phase (③) nicht mit der Prozessabwasser-Phase im Sammelbehälter 5 vereinigt. Vielmehr wird die Prozessabwasser-Phase ohne vorherige Vereinigung mit der Alkali-Salze beinhaltenden Nebenkomponenten-Phase (③) der Rektifikationseinheit 6 zur Abtrennung des noch enthaltenen n-Butanols zugeführt.

Das in der Rektifkationseinheit 6 erhaltene wässrige Sumpfprodukt ist nahezu frei von organischen Bestandteilen und kann vorteilhafterweise der Entsorgung in einer Kläranlage zugeführt werden. Die in der Extraktionseinheit 8 erhaltene, Alkali-Salze beinhaltenden Nebenkomponenten-Phase (③) kann gegebenenfalls unmittelbar einer Verbrennungseinheit zugeführt werden.

Gemäß der Figur 3 ist es jedoch vorteilhaft, diese Alkali-Salze beinhaltenden Nebenkomponenten-Phase (③) nicht unmittelbar einer Verbrennungseinheit zuzuführen, sondern zunächst in einer Verdampfungseinheit 9 einer zuzuführen, um zumindest einen Teil des in dieser Alkali-Salze beinhaltenden Nebenkomponenten-Phase (③) enthaltenen Wassers abzutrennen. Das auf diese Weise abgetrennte Wasser kann ebenfalls unmittelbar einer Kläranlage zugeführt werden.

Die in der Verdampfungsseinheit 9 erhaltene, wasserarme Alkali-Salze beinhaltende Nebenkomponenten-Phase kann dann gegebenenfalls einer weiteren Aufreinigungseinheit 10 zugeführt werden, in der beispielsweise mittels Umkehrosmose oder Elektrodialyse noch immer enthaltenes n-Butanol sowie gegebenenfalls noch immer enthaltenes Wasser abgetrennt werden. Die dabei zurückbleibende, salzhaltige organische Phase kann dann der Verbrennung zugeführt werden, während der abgetrennte n-Butanol (⑤) gegebenenfalls in die Rektifikationseinheit 3 zurückgeführt wird.

### BEZUGSZEICHENLISTE

- 1,2: Kaskadierte Reaktionsbereiche der Reaktionszone
- 3: Rektifikationskolonne
- 4: Abscheider
- 5: Sammelbehälter
- 6: Rektifikationskolonne zur Abtrennung des n-Butanols
- 7: Extraktionseinheit A
- 8: Extraktionseinheit B
- 9: Verdampfungsseinheit
- 10: Abtrennvorrichtung (Umkehrosmose oder Elektrodialyse)

## Patentansprüche

1. Ein Verfahren zur Herstellung einer aufgereinigten (Meth)Acrylsäurealkylester-Phase, beinhaltend die Verfahrensschritte:
I) Umsetzung einer Alkohol-Phase mit einer (Meth)Acrylsäure-Phase in Gegenwart eines Veresterungskatalysators unter Erhalt einer (Meth)Acrylsäurealkylester-Phase;
II) Destillative Abtrennung zumindest eines Teils des in der (Meth)Acrylsäurealkylester-Phase enthaltenen Wassers unter Erhalt
- einer wasserarmen, den (Meth)Acrylsäurealkylester beinhaltenden (Meth)Acrylsäurealkylester-Phase, sowie
- eines wässrigen Kopfproduktes;
III) Trennung des wässrigen Kopfproduktes in eine organische Phase und eine Prozessabwasser-Phase;
IV) weitere Aufreinigung der wasserarmen, den (Meth)Acrylsäure-alkylester beinhaltenden (Meth)Acrylsäurealkylester-Phase durch ein Aufreinigungsverfahren beinhaltend einen alkalische Reaktivextraktions-Schritt unter Erhalt
- einer aufgereinigten (Meth)Acrylsäurealkylester-Phase sowie
- einer Alkali-Salze beinhaltenden Nebenkomponenten-Phase;
V) Befreien der aufgereinigten (Meth)Acrylsäurealkylester-Phase von weiteren Nebenprodukten unter Erhalt einer hochreinen (Meth)Acrylsäurealkylester-Phase;
wobei mindestens eine der folgenden Alternativen i) oder ii), vorzugsweise i) und ii) zutrifft
i) die Alkali-Salze beinhaltende Nebenkomponenten-Phase wird einer Verbrennungseinheit zugeführt, wobei die Alkali-Salze beinhaltende Nebenkomponenten-Phase vor der Zuführung in die Verbrennungseinheit mit maximal 50 Gew.-%/Stunde, bezogen auf die pro Stunde anfallende Menge an Alkali-Salzen beinhaltende Nebenkomponenten-Phase, der Prozessabwasser-Phase vereinigt wird;
ii) die Prozessabwasser- Phase wird, nach Abtrennung mindestens eines Teils des in der Prozessabwasser-Phase noch enthaltenen, nicht umgesetzten Alkohols, der Reinigung in einer Kläranlage zugeführt, wobei die Prozessabwasser-Phase vor der Zuführung in die Kläranlage mit maximal 50 Gew.-%/Stunde, bezogen auf die pro Stunde anfallende Menge an Prozessabwasser-Phase, mit der die Alkali-Salze beinhaltende Nebenkomponenten-Phase vereinigt wird.

2. Das Verfahren nach Anspruch 1, wobei die Alkohol-Phase n-Butanol und die (Meth)Acrylsäure-Phase Acrylsäure beinhaltet.

3. Das Verfahren nach einem der vorhergehenden Ansprüche, wobei aus der Alkali-Salze beinhaltenden Nebenkomponenten-Phase vor deren Zuführung in die Verbrennungseinheit mindestens ein Teil des in dieser Nebenkomponenten-Phase noch enthaltenen Wassers unter Erhalt einer wasserarmen, Alkali-Salze beinhaltenden Nebenkomponenten-Phase abgetrennt wird.

4. Das Verfahren nach Anspruch 3, wobei dieses Abtrennen durch Verdampfen erfolgt.

5. Das Verfahren nach einem der Ansprüche 1 bis 4, wobei aus der Alkali-Salze beinhaltenden Nebenkomponenten-Phase oder der wasserarmen, Alkali-Salze beinhaltenden Nebenkomponenten-Phase mindestens ein Teil des in dieser wasserarmen Nebenkomponenten-Phase noch enthaltenen, nicht umgesetzten Alkohols und gegebenenfalls des noch enthaltenen Wassers abgetrennt wird.

6. Das Verfahren nach Anspruch 5, wobei dieses Abtrennen durch Elektrodialyse oder Umkehrosmose erfolgt.

7. Das Verfahren nach Anspruch 5, wobei der abgetrennte, nicht umgesetzte Alkohol in den Verfahrensschritt I) oder II) zurückgeführt wird.

8. Das Verfahren nach einem der vorhergehenden Ansprüche, wobei der gemäß der Alternative ii) abgetrennte, nicht umgesetzte Alkohol in den Verfahrenschritt I) oder II) zurückgeführt wird.

9. Das Verfahren nach einem der vorhergehenden Ansprüche, wobei das im Verfahrensschritt II) erhaltene, organische Sumpfprodukt im Verfahrensschritt IV) vor der alkalischen Reaktivextraktion mit Wasser gewaschen wird.

10. Das Verfahren nach einem der vorhergehenden Ansprüche, wobei die wasserarme (Meth)Acrylsäurealkylester-Phase vor der alkalischen Reaktivextraktion
(α1) 70 bis 90 Gew.-% des (Meth)Acrylsäurealkylesters,
(α2) 5 bis 20 Gew.-% des nicht umgesetzten Alkohols,
(α3) 0,1 bis 5 Gew.-% der nicht umgesetzten (Meth)Acrylsäure,
(α4) 0,1 bis 10 Gew.-% Wasser, sowie
(α5) 1 bis 10 Gew.-% von den Komponenten (α1) bis (α4) verschiedene Verunreinigungen,
beinhaltet, wobei die Summe der Komponenten (α1) bis (α5) 100 Gew.-% beträgt.

11. Das Verfahren nach einem der vorhergehenden Ansprüche, wobei die Alkali-Salze beinhaltende Nebenkomponenten-Phase
(β1) 60 bis 90 Gew.-% Wasser,
(β2) 0,1 bis 5 Gew.-% des nicht umgesetzten Alkohols,
(β3) 5 bis 30 Gew.-% des Alkali-Salzes der nicht umgesetzten (Meth)Acrylsäure,
(β4) 0,1 bis 10 Gew.-% von den Komponenten (β1) bis (β3) verschiedene Verunreinigungen,
beinhaltet, wobei die Summe der Komponenten (β1) bis (β4) 100 Gew.% beträgt.

12. Das Verfahren nach einem der vorhergehenden Ansprüche, wobei die aufgereinigte (Meth)Acrylsäurealkylester-Phase zu mindestens 77,5 Gew.-%, bezogen auf das Gesamtgewicht der (Meth)Acrylsäurealkylester-Phase, auf dem (Meth)Acrylsäurealkylester basiert.

13. Ein Verfahren zur Herstellung eines auf (Meth)Acrylsäurealkylestern basierenden Polymers, beinhaltend die Verfahrensschritte
(a) Herstellung einer hochreinen (Meth)Acrylsäurealkylester-Phase nach einem der Ansprüche 1 bis 12;
(b) radikalische Polymerisation des (Meth)Acrylsäurealkylesters.

## Claims

1. Process for producing a purified alkyl (meth)acrylate phase, which comprises the process steps:
I) reaction of an alcohol phase with a (meth)acrylic acid phase in the presence of an esterification catalyst to give an alkyl (meth)acrylate phase,
II) removal of at least part of the water present in the alkyl (meth)acrylate phase by distillation to give
- a low-water alkyl (meth)acrylate phase comprising the alkyl (meth)acrylate and
- an aqueous overhead product;
III) separation of the aqueous overhead product into an organic phase and a process wastewater phase;
IV) further purification of the low-water alkyl (meth)acrylate phase comprising the alkyl (meth)acrylate by a purification process comprising an alkaline reactive extraction step to give
- a purified alkyl (meth)acrylate phase and
- a secondary component phase comprising alkali metal salts;
V) freeing of the purified alkyl (meth)acrylate phase of further by-products to give a high-purity alkyl (meth)acrylate phase;
where at least one of the following alternatives i) or ii), preferably i) and ii), applies
i) the secondary component phase comprising alkali metal salts is fed to an incineration unit, where the secondary component phase comprising alkali metal salts is combined with a maximum of 50% by weight/hour, based on the amount of secondary component phase comprising alkali metal salts obtained per hour, of the process wastewater phase before being fed into the incineration unit;
ii) the process wastewater phase is, after removal of at least part of the unreacted alcohol still present in the process wastewater phase, passed to purification in a water treatment plant, where the process wastewater phase is combined with a maximum of 50% by weight/hour, based on the amount of process wastewater phase obtained per hour, of the secondary component phase comprising alkali metal salts before being fed to the water treatment plant.

2. Process according to Claim 1, wherein the alcohol phase comprises n-butanol and the (meth)acrylic acid phase comprises acrylic acid.

3. The process according to either of the preceding claims, wherein at least part of the water still present in the secondary component phase comprising alkali metal salts is separated off from the secondary component phase to give a low-water secondary component phase comprising alkali metal salts before this phase is fed into the incineration unit.

4. Process according to Claim 3, wherein this separation is effected by evaporation.

5. Process according to any of Claims 1 to 4, wherein at least part of the unreacted alcohol still present in the low-water secondary component phase and optionally of the water still present is separated off from the secondary component phase comprising alkali metal salts or the low-water secondary component phase comprising alkali metal salts.

6. Process according to Claim 5, wherein this separation is effected by electrodialysis or reverse osmosis.

7. Process according to Claim 5, wherein the unreacted alcohol which has been separated off is recirculated to process step I) or II).

8. Process according to any of the preceding claims, wherein the unreacted alcohol which has been separated off according to alternative ii) is recirculated to process step I) or II).

9. Process according to any of the preceding claims, wherein the organic bottom product obtained in process step II) is scrubbed with water before the alkaline reactive extraction in process step IV).

10. Process according to any of the preceding claims, wherein the low-water alkyl (meth)acrylate phase before the alkaline reactive extraction comprises
(α1) from 70 to 90% by weight of the alkyl (meth)acrylate,
(α2) from 5 to 20% by weight of the unreacted alcohol,
(α3) from 0.1 to 5% by weight of the unreacted (meth) acrylic acid,
(α4) from 0.1 to 10% by weight of water and
(α5) from 1 to 10% by weight of impurities different from the components (α1) to (α4), where the sum of the components (α1) to (α5) is 100% by weight.

11. Process according to any of the preceding claims, wherein the secondary component phase comprising alkali metal salts comprises
(β1) from 60 to 90% by weight of water,
(β2) from 0.1 to 5% by weight of the unreacted alcohol,
(β3) from 5 to 30% by weight of the alkali metal salts of the unreacted (meth)acrylic acid,
(β4) from 0.1 to 10% by weight of impurities different from the components (β1) to (β3),
where the sum of the components (β1) to (β4) is 100% by weight.

12. Process according to any of the preceding claims, wherein the purified alkyl (meth)acrylate phase is based to an extent of at least 77.5% by weight, based on the total weight of the alkyl (meth)acrylate phase, on the alkyl (meth)acrylate.

13. Process for producing a polymer based on alkyl (meth)acrylates, which comprises the process steps
(a) production of a high-purity alkyl (meth)acrylate phase according to any of Claims 1 to 12;
(b) free-radical polymerization of the alkyl (meth)acrylate.

## Revendications

1. Procédé pour la préparation d'une phase purifiée d'ester alkylique de l'acide (méth)acrylique, comportant les étapes de procédé :
I) transformation d'une phase d'alcool avec une phase d'acide (méth)acrylique en présence d'un catalyseur d'estérification avec obtention d'une phase d'ester alkylique de l'acide (méth)acrylique ;
II) séparation par distillation d'au moins une partie de l'eau contenue dans la phase d'ester alkylique de l'acide (méth)acrylique avec obtention
- d'une phase, pauvre en eau, comportant l'ester alkylique de l'acide (méth)acrylique ainsi que
- d'un produit de tête aqueux ;
III) séparation du produit de tête aqueux en une phase organique et une phase d'eau résiduaire de procédé ;
IV) nouvelle purification de la phase, pauvre en eau, d'ester alkylique de l'acide (méth)acrylique comportant l'ester alkylique de l'acide (méth)acrylique par un procédé de purification comportant une étape d'extraction réactive alcaline, avec obtention
- d'une phase d'ester alkylique de l'acide (méth)acrylique purifiée ainsi que
- d'une phase de composants secondaires comportant des sels alcalins ;
V) libération de la phase d'ester alkylique de l'acide (méth)acrylique purifiée d'autres produits secondaires avec obtention d'une phase d'ester alkylique de l'acide (méth)acrylique de haute pureté ;
au moins une des alternatives suivantes i) ou ii), de préférence i) et ii) étant d'application
i) la phase de composants secondaires comportant des sels alcalins est introduite dans une unité de combustion, la phase de composants secondaires comportant des sels alcalins étant réunie, avant l'introduction dans l'unité de combustion, avec au maximum 50% en poids/heure, par rapport à la quantité produite par heure de phase de composants secondaires comportant des sels alcalins, de la phase d'eau résiduaire de procédé ;
ii) la phase d'eau résiduaire de procédé est introduite, après séparation d'au moins une partie de l'alcool non transformé encore contenu dans la phase d'eau résiduaire de procédé dans l'étape de purification d'une installation de clarification, la phase d'eau résiduaire de procédé étant réunie, avant l'introduction dans l'installation de clarification avec au maximum 50% en poids/heure, par rapport à la quantité produite par heure de phase d'eau résiduaire de procédé, de la phase de composants secondaires comportant des sels alcalins.

2. Procédé selon la revendication 1, la phase d'alcool comportant du n-butanol et la phase d'acide (méth)acrylique comportant de l'acide acrylique.

3. Procédé selon l'une quelconque des revendications précédentes, en séparant, de la phase de composants secondaires comportant des sels alcalins, avant son introduction dans l'unité de combustion, au moins une partie de l'eau encore contenue dans cette phase de composants secondaires avec obtention d'une phase de composants secondaires pauvre en eau, comportant des sels alcalins.

4. Procédé selon la revendication 3, cette séparation ayant lieu par évaporation.

5. Procédé selon l'une quelconque des revendications 1 à 4, en séparant, de la phase de composants secondaires comportant des sels alcalins ou de la phase de composants secondaires pauvre en eau, comportant des sels alcalins, au moins une partie de l'alcool non transformé encore contenu dans cette phase de composants secondaires pauvre en eau et le cas échéant l'eau encore contenue.

6. Procédé selon la revendication 5, cette séparation ayant lieu par électrodialyse ou osmose inverse.

7. Procédé selon la revendication 5, l'alcool non transformé, séparé étant recyclé dans l'étape de procédé I) ou II).

8. Procédé selon l'une quelconque des revendications précédentes, l'alcool non transformé, séparé selon l'alternative ii) étant recyclé dans l'étape de procédé I) ou II).

9. Procédé selon l'une quelconque des revendications précédentes, le produit de fond organique, obtenu dans l'étape de procédé II) étant lavé dans l'étape de procédé IV), avant l'extraction réactive alcaline, avec de l'eau.

10. Procédé selon l'une quelconque des revendications précédentes, la phase d'ester alkylique de l'acide (méth)acrylique pauvre en eau comportant, avant l'extraction réactive alcaline
(α1) 70 à 90% en poids d'ester alkylique de l'acide (méth)acrylique,
(α2) 5 à 20% en poids d'alcool non transformé,
(α3) 0,1 à 5% en poids d'acide (méth)acrylique non transformé,
(α4) 0,1 à 10% en poids d'eau ainsi que
(α5) 1 à 10% en poids d'impuretés différentes des composants (α1) à (α4),
la somme des composants (α1) à (α5) valant 100% en poids.

11. Procédé selon l'une quelconque des revendications précédentes, la phase de composants secondaires comportant des sels alcalins comportant
(ß1) 60 à 90% en poids d'eau,
(ß2) 0,1 à 5% en poids d'alcool non transformé,
(ß3) 5 à 30% en poids du sel alcalin de l'acide (méth)acrylique non transformé,
(ß4) 0,1 à 10% en poids d'impuretés différentes des composants (ß1) à (ß3),
la somme des composants (ß1) à (ß4) valant 100% en poids.

12. Procédé selon l'une quelconque des revendications précédentes, la phase d'ester alkylique de l'acide (méth)acrylique purifiée représentant au moins 77,5% en poids, par rapport au poids total de la phase d'ester alkylique de l'acide (méth)acrylique, sur base de l'ester alkylique de l'acide (méth)acrylique.

13. Procédé pour la préparation d'un polymère à base d'esters alkyliques de l'acide (méth)acrylique, comportant les étapes de procédé :
(a) préparation d'une phase d'ester alkylique de l'acide (méth)acrylique de haute pureté selon l'une quelconque des revendications 1 à 12 ;
(b) polymérisation radicalaire de l'ester alkylique de l'acide (méth)acrylique.
